(19) Europäisches Patentamt / European Patent Office / Office européen des brevets

(11) **EP 4 137 507 A1**

(12) **EUROPEAN PATENT APPLICATION**
published in accordance with Art. 153(4) EPC

(43) Date of publication:
**22.02.2023 Bulletin 2023/08**

(21) Application number: **21789246.2**

(22) Date of filing: **31.03.2021**

(51) International Patent Classification (IPC):
*C07K 14/47* [(2006.01)]    *C07K 14/765* [(2006.01)]
*A61K 38/00* [(2006.01)]    *A61P 43/00* [(2006.01)]
*A61K 38/38* [(2006.01)]

(52) Cooperative Patent Classification (CPC):
**A61K 38/00; A61K 38/38; A61P 43/00;**
**C07K 14/47; C07K 14/765**

(86) International application number:
**PCT/KR2021/003959**

(87) International publication number:
**WO 2021/210812 (21.10.2021 Gazette 2021/42)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **14.04.2020 KR 20200044982**
**30.03.2021 KR 20210040819**

(71) Applicant: **Senelix Co., Ltd.**
**Seoul 05836 (KR)**

(72) Inventor: **OH, Jun Seo**
**Seoul 03709 (KR)**

(74) Representative: **Mathys & Squire**
**The Shard**
**32 London Bridge Street**
**London SE1 9SG (GB)**

Remarks:
The complete document including Reference
Table(s) and the Sequence Listing(s) can be
downloaded from the EPO website

(54) **RECOMBINANT FUSION PROTEIN FOR PREVENTING OR TREATING FIBROTIC DISEASES**

(57)    The present disclosure relates to a fusion protein of albumin and a retinol-binding protein, which can be used to prevent or treat fibrotic diseases occurring in the liver, pancreas, lungs, etc. The fusion protein of the present disclosure inhibits the activation of stellate cells, which causes tissue fibrosis, or induces the deactivation thereof, thus enabling the prevention or treatment of fibrotic diseases, and exhibits a more potent effect at a low concentration as compared to existing fusion proteins for the same purpose. Thus, the fusion protein is expected to be a general-purpose platform technology capable of remarkably reducing the dose and frequency of administration of protein therapeutic agents to the human body.

FIG. 4B

**Description**

[Technical Field]

The present disclosure relates to a fusion protein of albumin and a retinol-binding protein, which can be used to prevent or treat fibrotic diseases occurring in the liver, pancreas, lungs, etc.

[Background Art]

Tissue fibrosis leads to a fatal result of the failure of tissue function. For example, liver fibrosis results in the failure of liver function, followed by cirrhosis and cancer, and fibrous tissues are observed in both chronic pancreatitis and pancreatic cancer. No therapeutic agent for fibrotic diseases is available at present, and tissue transplantation is employed as the most promising technique for treatment. The molecular mechanism of tissue fibrosis has not been elucidated clearly yet, and research and development are necessary for therapeutic agents for fibrotic diseases.

Recently, it was found out that tissue fibrosis occurs during the activation and transdifferentiation of stellate cells that constitute tissues. Activated stellate cells overexpress extracellular matrices such as collagen and differentiate into myofibroblasts. The activated stellate cells play a decisive role in the onset of tissue fibrosis.

Stellate cells are distributed not only in the liver but also in the pancreas, lungs, kidneys, intestines, etc. and play a central role in the regulation of retinoid homeostasis throughout the body. Food-derived vitamin A (retinol) circulates in the bloodstream as being bound to a retinol-binding protein (RBP) and is transported into stellate cells by the RBP receptor STRA6. Then, it is stored as retinyl ester in lipid droplets in the cytoplasm. Meanwhile, it is known that albumin inhibits the activation of stellate cells and the expression of albumin in activated stellate cells deactivates the cells.

In the previous research, the inventors of the present disclosure have prepared a fusion protein which targets stellate cells, wherein RBP that can be transported into stellate cells by STRA6 is bound to albumin, in order to inhibit the activation of stellate cells or deactivated the cells and have identified its effect on the prevention and treatment of fibrosis (KR 10-1395394).

**[Disclosure]**

[Technical Problem]

The present disclosure has been made to improve the effect of inhibiting the activation of stellate cells or deactivating the cells of the existing RBP-albumin fusion protein and is directed to providing an RBP-albumin fusion protein wherein one or more amino acid sequence of albumin is substituted and RBP-albumin fusion proteins with different arrangements.

The present disclosure is also directed to providing a method for producing the recombinant protein of the present disclosure in large scale by providing a vector capable of expressing the fusion protein and a transformant in which the vector is introduced.

The present disclosure is also directed to providing a composition containing the fusion protein for preventing or treating a fibrotic disease since the fusion protein exhibits better effect of inhibiting the activation of stellate cells or deactivating the cells than the existing RBP-albumin fusion protein.

However, the technical problems to be solved by the present disclosure are not limited to those mentioned above and other problems not mentioned above will be clearly understood by those having ordinary skill in the art from the following description.

[Technical Solution]

The present disclosure provides a fusion protein consisting of or containing an amino acid sequence of SEQ ID NO 1 or 2.

The present disclosure also provides a fusion protein consisting of or containing any amino acid sequence of SEQ ID NOS 3-8.

In an exemplary embodiment of the present disclosure, the fusion protein may be for preventing or treating tissue fibrosis.

The present disclosure also provides a polynucleotide encoding the fusion protein described above. The polynucleotide may consist of or contain any base sequence of SEQ ID NOS 9-14.

The present disclosure also provides a recombinant vector containing a gene encoding the fusion protein, and a transformant including the recombinant vector.

The present disclosure also provides a method for producing a fusion protein, which includes: (1) a step of preparing a recombinant vector containing a gene encoding a fusion protein containing any amino acid sequence of SEQ ID NOS 3-8; (2) a step of preparing a transformant by introducing the recombinant vector into a host cell; (3) a step of culturing the transformant; and (4) a step of obtaining a fusion protein from the cultured transformant.

In an exemplary embodiment of the present disclosure, the gene encoding the fusion protein may contain a polynucleotide containing one or more base sequence selected from a group consisting of SEQ ID NOS 9-14.

In another exemplary embodiment of the present disclosure, the host cell may be a mammalian cell, specifically a mammalian cancer cell, more specifically a CHO cell.

The present disclosure may also provide a pharmaceutical composition for preventing or treating a fibrotic disease, which contains a fusion protein consisting of any amino acid sequence of SEQ ID NOS 1-8.

The present disclosure may also provide a method for preventing or treating a fibrotic disease, which includes a step of administering a fusion protein consisting of any amino acid sequence of SEQ ID NOS 1-8 to a subject.

In an exemplary embodiment of the present disclosure, the subject may be a human in need of prevention or treatment of a fibrotic disease, particularly a patient with a non-alcoholic fatty liver disease.

In another exemplary embodiment of the present disclosure, the method may further include a step of diagnosing the degree of fibrosis after the administration of the fusion protein by conducting CT imaging or biopsy.

In an exemplary embodiment of the present disclosure, the fibrotic disease may be one or more disease selected from a group consisting of liver fibrosis, chronic hepatitis, cirrhosis, hepatic cancer, chemotherapy-associated steatohepatitis (CASH), lung fibrosis, renal fibrosis, renal failure, pancreatic fibrosis, chronic pancreatitis and pancreatic cancer.

The present disclosure also provides a use of the fusion protein described above for preparation of a medication for preventing or treating a fibrotic disease.

[Advantageous Effects]

A fusion protein of the present disclosure inhibits the activation of stellate cells, which causes tissue fibrosis, or induces the deactivation thereof, thus enabling the prevention or treatment of fibrotic diseases, and exhibits a more potent effect at a low concentration as compared to existing fusion proteins for the same purpose. Thus, the fusion protein is expected to be a general-purpose platform technology capable of remarkably reducing the dose and frequency of administration of protein therapeutic agents to the human body.

[Brief Description of Drawings]

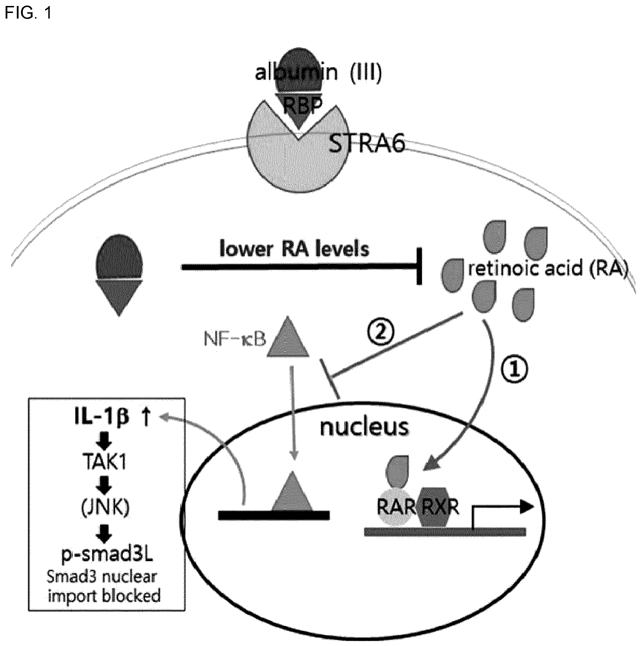

FIG. 1 shows a mechanism by which a fusion protein wherein albumin III is bound to RBP acts as being included in an activated stellate cell.
FIG. 2 shows a result of comparing the effect of inducing the deactivation of activated stellate cells of fusion proteins 1 and 2 of the present disclosure and existing fusion proteins (RBP-albumin III and albumin III-RBP).
FIG. 3 shows a result of comparing the effect of inducing the deactivation of activated stellate cells of fusion proteins 1 and 2 of the present disclosure with fusion proteins 3-6.
FIG. 4A and FIG. 4B show a result of comparing the degree of liver fibrosis in an animal model of liver fibrosis depending on the administration of fusion proteins 1 and 4 and RBP-albumin through sirius red staining.

[Best Mode]

Albumin is a multifunctional plasma protein synthesized primarily by liver cells. Albumin has three domains, each formed by two subdomains A and B. It is known that albumin binds to various hydrophobic substances including fatty acids and retinoic acid and transports the molecules in blood. According to crystallographic analysis, five strong fatty acid-binding sites are distributed asymmetrically in albumin (one in subdomain IB, one between IA and IIA, two in IIIA, and one in IIIB).

In the previous research, the inventors of the present disclosure have prepared a fusion protein wherein albumin and a retinol-binding protein (RBP) for targeting stellate cells are bound, and confirmed that, after being introduced into activated stellate cells, the fusion protein deactivates the cells and thus enables prevention and treatment of fibrotic diseases. In addition, the inventors of the present disclosure have found out that retinoic acid (RA) plays an important role during activation of stellate cells and the fusion protein controls the activation of stellate cells by reducing the intracellular level of RA. Therefore, in order to develop a fusion protein that can inhibit the activation of stellate cells or induce the deactivation thereof through more stable binding to retinoic acid (RA), they have prepared new albumin-RBP fusion proteins using sequence variations of albumin and arrangements of albumin sub-domains and RBP of various combinations.

In the previous research, the domain III of albumin has shown the best effect of inhibiting the activation of stellate cells or inducing the deactivation thereof in the fusion proteins. Based on this result, the inventors of the present disclosure have intended to prepare a recombinant fusion protein with enhanced binding ability to retinoic acid.

Thus, presuming that the 526th and 600th bulky amino acids of domain III in the existing fusion protein of albumin domain III and RBP interfere with binding to RA, they have substituted them with smaller amino acids and investigated their effect of deactivating activated stellate cells through binding with RA (see SEQ ID NOS 1 and 2).

Specifically, the inventors of the present disclosure have designed fusion proteins 1 and 2 as follows by substituting the 526th amino acid residue (phenylalanine) and the 600th amino acid residue (valine) of albumin domain III with valine and alanine, respectively.

- Fusion protein 1:

RBP (1-193) + albumin III {404-526(F→V)-600(V→A)-601}

- Fusion protein 2:

- Fusion protein 2: albumin [signal peptide (1-18) + III {404-526(F→V)-600(V→A)-601}] + RBP (17-192)

In addition, the inventors of the present disclosure have identified that the sub-domains IB and IIIA of albumin bind very stably to retinoic acid and have designed fusion proteins 3-6 as follows.

- Fusion protein 3:

RBP (1-193) + albumin [IIIA {404-517(V)} + IB (131-218)]

- Fusion protein 4:

RBP (1-193) + albumin [IIIA {404-517(V)} + IB (134-218)]

- Fusion protein 5:

- Fusion protein 5: albumin [signal peptide (1-18) + IIIA {404-517(V)} + IB (131-218)] + RBP (17-192)

- Fusion protein 6:

- Fusion protein 6: albumin [signal peptide (1-18) + IIIA {404-517(V)} + IB (134-218)] + RBP (17-192)

Then, the inventors of the present disclosure treated activated stellate cells with the fusion protein 1 or 2 and compared the effect of deactivating the stellate cells with previously developed fusion proteins (RBP-albumin III and albumin III-RBP) by measuring the expression level of α-SMA (α-smooth muscle actin) and collagen type I, which are markers of activated stellate cells. As a result, it was confirmed that the fusion proteins 1 and 2 of the present disclosure can significantly reduce the expression of α-SMA and collagen I at lower concentrations as compared to the previously developed fusion proteins (see Example 1).

Through this, the inventors of the present disclosure have identified that the substitution of the 526th and 600th amino acids of albumin III with amino acids of smaller molecular weights can more effectively induce the deactivation of stellate cells due to increased binding ability to retinoic acid.

Therefore, the fusion protein of the present disclosure wherein albumin III with the 526th and 600th amino acids substituted independently and RBP are bound can be used for treatment of fibrotic diseases. In the fusion protein, the amino acid at the 526th amino acid residue of albumin III may be substituted with valine, alanine or glycine, and the amino acid at the 600th amino acid residue may be substituted with alanine or glycine. The fusion protein may consist of or contain an amino acid sequence of SEQ ID NO 1 or 2.

Then, the inventors of the present disclosure compared the effect of inhibiting the expression of α-SMA and collagen I of the fusion proteins 1 and 2, which showed superior effect of inhibiting the activation of stellate cells as compared to the existing fusion proteins, with fusion proteins 3-6. As a result, it was confirmed that the fusion proteins 3-6 can effectively inhibit the activation of stellate cells at lower concentrations (Example 2).

In addition, the inventors of the present disclosure investigated the effect of ameliorating liver fibrosis of the fusion proteins 1 and 4 and the existing fusion protein (RBP-albumin III) using a carbon tetrachloride-induced liver fibrosis animal model. As a result, it was confirmed that the fusion proteins 1 and 4 can ameliorate fibrosis more effectively at lower concentrations as compared to the RBP-albumin III (Example 3).

Therefore, the present disclosure may provide a pharmaceutical composition for preventing or treating a fibrotic disease, which contains one or more fusion protein selected from a group consisting of the fusion proteins 1-6, and may provide a method for preventing or treating a fibrotic disease, which includes a step of administering two or more fusion proteins selected from a group consisting of the fusion proteins 1-6 to a subject.

Since it was confirmed that the fusion proteins 1-6 of the present disclosure can induce the deactivation of activated stellate cells more effectively at lower concentrations as compared to the existing RBP-albumin fusion proteins, it is expected that the pharmaceutical composition of the present disclosure can be a general-purpose platform technology capable of remarkably reducing the dose and frequency of administration of protein therapeutic agents to the human body.

In the present disclosure, the "fibrotic disease" refers to a disease caused by fibrosis whereby normal tissue is damaged and replaced by fibrous connective tissue, resulting in malfunction of an organ, and includes any disease wherein a part of an organ is hardened due to fibrosis without limitation, such as liver fibrosis, chronic hepatitis, cirrhosis, hepatic cancer, chemotherapy-associated steatohepatitis (CASH), lung fibrosis, renal fibrosis, renal failure, pancreatic fibrosis, chronic pancreatitis and pancreatic cancer.

In the present disclosure, the "subject" may be any mammal without limitation. Specifically, it may be human or livestock.

In the present disclosure, prevention refers to any action of delaying the onset of a fibrotic disease by administering the pharmaceutical composition according to the present disclosure, and treatment refers to any action of ameliorating or favorably changing the symptoms of a fibrotic disease by administering the pharmaceutical composition according to the present disclosure.

In the present disclosure, the albumin used to form the fusion protein may be derived from any species. But, specifically, it may be one derived from the same species as the subject to which it is administered in order to avoid the risk of immunogenicity.

In the present disclosure, the pharmaceutical composition may further contain, in addition to the fusion proteins 1-6, one or more substance known to be capable of preventing or treating tissue fibrosis, and may further contain a suitable carrier, excipient and diluent commonly used to prepare a pharmaceutical composition.

In the present disclosure, the "carrier" is also called a vehicle and refers to a compound that facilitates the delivery of a protein or a peptide into a cell or a tissue. For example, dimethyl sulfoxide (DMSO) is a carrier commonly used to facilitate the delivery of many organic substances into the cell or tissue of an organism.

In the present disclosure, the "diluent" is defined as a compound diluted in water in which a protein or a peptide is dissolved in order to stabilize the biologically active form of the protein or peptide. Salts dissolved in buffer solutions are used as diluents in the art. Phosphate-buffered saline is a commonly used buffer solution, because it mimics the salt state of human body fluid. Since the buffer salt can control the pH of a solution at low concentration, the buffer diluent rarely modifies the biological activity of a compound. Compounds including azelaic acid may be administered to a human patient either as they are or in combination with other ingredients or suitable carriers or excipients used in combination therapy.

In addition, the pharmaceutical composition according to the present disclosure for preventing or treating a fibrotic disease may be formulated as a powder, a granule, a tablet, a capsule, a suspension, an emulsion, a syrup, a formulation for external use such as an aerosol, etc. or a sterile injection solution according to a conventional method. The composition of the present disclosure may be administered orally or parenterally (e.g., intravenously, subcutaneously, intraperitoneally or topically) depending on purposes. The administration dosage will vary depending on the patient's condition and body weight, the severity of a disease, the type of the formulation, administration route and administration period but may be determined adequately by those skilled in the art. For example, about 0.001-1000 mg may be administered in combination with a pharmaceutically acceptable carrier. The composition of the present disclosure may be administered once or several times a day, if necessary, either alone or in combination with surgery, hormone therapy, medication and use of biological response modifiers.

Meanwhile, a protecting group such as an acetyl group, a fluorenylmethoxycarbonyl group, a formyl group, a palmitoyl group, a myristyl group, a stearyl group, polyethylene glycol (PEG), etc. may be bound to the amino terminal of the recombinant fusion protein of the present disclosure, and the carboxyl terminal of the peptide may be modified with a hydroxyl group (-OH), an amino group ($-NH_2$), an azide group ($-NHNH_2$), etc.

In addition, a fatty acid, an oligosaccharide chain, nanoparticles (gold particles, liposomes, heparin, hydrogels, etc.), an amino acid, a carrier protein, etc. may be bound to the terminal or amino acid R-residue of the fusion protein of the present disclosure. Such amino acid modification improves the potency and stability of the protein of the present disclosure.

In the present disclosure, the term "stability" refers to not only in-vivo stability but also storage stability (at room temperature or during storage in a freezer).

The present disclosure also provides a method for producing the fusion protein described above, which includes:

(1) a step of preparing a recombinant vector containing a gene encoding a fusion protein containing any amino acid sequence of SEQ ID NOS 3-8;
(2) a step of preparing a transformant by introducing the recombinant vector into a host cell;
(3) a step of culturing the transformant; and
(4) a step of obtaining a fusion protein from the cultured transformant.

In the present disclosure, the "gene" should be understood as the broadest meaning, which encodes a structural protein or a regulatory protein, and is not included as long as it is a DNA fragment encoding the fusion proteins 1-6 of the present disclosure. Specifically, it may contain any base sequence of SEQ ID NOS 9-14.

Also, in the present disclosure, the "vector" refers to a DNA construct containing a DNA sequence which is operably linked to a suitable control sequence capable of expressing a DNA in a suitable host. The vector may be a plasmid, a phage particle or simply a potential genome insert. When transformed into a suitable host, the vector may replicate and function independently of the host genome, or may be integrated into the genome of the host in some cases. Because a plasmid is the most commonly used type of a vector, the terms plasmid and vector are used sometimes interchangeably in the present disclosure.

A base sequence is "operably linked" when it is placed into a functional relationship with another base sequence. The base sequence may be a gene and a control sequence linked to be capable of expressing the gene when a suitable molecule (e.g., transcription-activating protein) binds to the control sequence. For example, a DNA for a pre-sequence or a secretory leader is operably linked to a DNA for a polypeptide if it is expressed as a pro-protein which participates in the secretion of the polypeptide; a promoter or an enhancer is operably linked to a coding sequence if it affects the transcription of the sequence; or a ribosome-binding site is operably linked to a coding sequence if it is positioned so as to facilitate translation. Generally, the "operably linked" means that the DNA sequences being linked are contiguous and, in the case of a secretory leader, contiguous and present in a reading frame. However, an enhancer is not necessarily contiguous. The linkage between the sequences is accomplished by ligation at convenient restriction enzyme sites. If such sites do not exist, a synthetic oligonucleotide adaptor or a linker is used in accordance with the conventional practice.

Also, in the present disclosure, "transformation" or "transfection" refers to the introduction of a DNA into a host for replication as an extrachromosomal factor or by chromosomal integration. In the present disclosure, the host cell used to prepare the fusion protein is a CHO cell, although not being limited thereto.

[Mode for Invention]

The present disclosure may be changed variously and may have various exemplary embodiments. Hereinafter, specific exemplary embodiments will be presented and described in detail. However, the present disclosure is not limited to the specific exemplary embodiments but should be understood to include all changes, equivalents and substitutes encompassed in the technical idea and scope of the present disclosure. In the following description of the present disclosure, details of known technologies may be omitted to avoid unnecessarily obscuring the present disclosure.

**[Experimental methods]**

**1. Isolation and culturing of hepatic stellate cells (HSCs)**

After perfusing the liver of 14-week-old male BALB/c mice with PBS (phosphate-buffered saline), followed by perfusion with GBSS (Gey's balanced salt solution) containing collagenase, pronase and DNase, the liver was extracted. After removing gall bladder and connective tissues attached to the liver and then placing a suspension of the hepatic cells in the same GBSS and treating at 37 °C for 12 minutes, centrifugation was performed at 1400 g for 20 minutes in a 13.4% Nycodenz gradient. Stellate cells were taken from the interface between the Nycodenz solution and an aqueous layer and cultured in DMEM (Dulbecco's modified Eagle's medium; Carlsbad, CA) supplemented with 10% fetal bovine serum (FBS). The purity of the stellate cells was evaluated by microscopic observation and western blotting using anti-tyrosine aminotransferase antibodies. When the cells were confluent, they were subcultured and used as activated stellate cells. The activation of the hepatic stellate cells was confirmed by morphological change and increased expression of α-SMA and collagen I.

## 2. Design and preparation of fusion protein

2-1. Design of fusion protein

Detailed information about fusion proteins 1-6 for enhancing binding ability to retinoic acid is shown in Table 1. The amino acids represented by lowercase letters indicate the sequence of a signal peptide, and the amino acids substituted in albumin III are bold-faced and underlined, and the underlined amino acids indicate RBP.

[Table 1]

| SEQ ID NO | Fusion protein | Amino acid sequence |
|---|---|---|
| 3 | Fusion protein 1: RBP (1-193) + albumin III {404-526 (F→V )-600 (V→A)-6 01} | mkwvwallllaawaaaERDCRVSSFRVKENFDKARFSGTWYAMAKKDPEGLFLQDNIVAEFSVDETGQMSATAKGRVRLLNNWDVCADMVGTFTDTEDPAKFKMKYWGVASFLQKGNDDHWIVDTDYDTYAVQYSCRLLNLDGTCADSYSFVFSRDPNGLPPEAQKIVRQRQEELCLARQYRLIVHNGYCDGRLVEEPQNLIKQNCELFEQLGEYKFQNALLVRYTKKVPEVSTPTLVEVSRNLGKVGSKCCKHPEAKRMPCAEDYLSVVLNQLCVLHEKTPVSDRVTKCCTESLVNRRPCFSALEVDETYVPKE**V**NAETFTFHADICTLSEKERQIKKQTALVELVKHPKATKEQLKAVMDDFAAFVEKCCKADDKETCFAEEGKKL**A**A |
| 4 | Fusion protein 2: albumin [signal peptide (1-18) + III {404~526(F→V)-600(V→A)-601}] + RBP (17-192) | mkwvtfisllflflfssaysLVEEPQNLIKQNCELFEQLGEYKFQNALLVRYTKKVPEVSTPTLVEVSRNLGKVGSKCCKHPEAKRMPCAEDYLSVVLNQLCVLHEKTPVSDRVTKCCTESLVNRRPCFSALEVDETYVPKE**V**NAETFTFHADICTLSEKERQIKKQTALVELVKHPKATKEQLKAVMDDFAAFVEKCCKADDKETCFAEEGKKL**A**AERDCRVSSFRVKENFDKARFSGTWYAMAKKDPEGLFLQDNIVAEFSVDETGQMSATAKGRVRLLNNWDVCADMVGTFTDTEDPAKFKMKYWGVASFLQKGNDDHWIVDTDYDTYAVQYSCRLLNLDGTCADSYSFVFSRDPNGLPPEAQKIVRQRQEELCLARQYRLIVHNGYCDG |

(continued)

| SEQ ID NO | Fusion protein | Amino acid sequence |
|---|---|---|
| 5 | Fusion protein 3: RBP (1-193) + albumin [IIIA {404-517(V)} + IB (131-218)] | MkwvwalllaawaaaERDCRVSSFRVKENFDKARFSGTWYAMAKKDPEGLFLQDNIVAEFSVDETGQMSATAKGRVRLLNNWDVCADMVGTFTDTEDPAKFKMKYWGVASFLQKGNDDHWIVDTDYDTYAVQYSCRLLNLDGTCADSYSFVFSRDPNGLPPEAQKIVRQRQEELCLARQYRLIVHNGYCDGRLVEEPQNLIKQNCELFEQLGEYKFQNALLVRYTKKVPEVSTPTLVEVSRNLGKVGSKCCKHPEAKRMPCAEDYLSVVLNQLCVLHEKTPVSDRVTKCCTESLVNRRPCFSALEVDDNPNLPRLVRPEVDVMCTAFHDNEETFLKKYLYEIARRHPYFYAPELLFFAKRYKAAFTECCQAADKAACLLPKLDELRDEGKASSA |
| 6 | Fusion protein 4: RBP (1-193) + albumin [IIIA {404-517(V)} + IB (134~218)] | MkwvwalllaawaaaERDCRVSSFRVKENFDKARFSGTWYAMAKKDPEGLFLQDNIVAEFSVDETGQMSATAKGRVRLLNNWDVCADMVGTFTDTEDPAKFKMKYWGVASFLQKGNDDHWIVDTDYDTYAVQYSCRLLNLDGTCADSYSFVFSRDPNGLPPEAQKIVRQRQEELCLARQYRLIVHNGYCDGRLVEEPQNLIKQNCELFEQLGEYKFQNALLVRYTKKVPEVSTPTLVEVSRNLGKVGSKCCKHPEAKRMPCAEDYLSVVLNQLCVLHEKTPVSDRVTKCCTESLVNRRPCFSALEVPNLPRLVRPEVDVMCTAFHDNEETFLKKYLYEIARRHPYFYAPELLFFAKRYKAAFTECCQAADKAACLLPKLDELRDEGKASSA |
| 7 | Fusion protein 5: albumin [signal peptide (1-18) + IIIA {404-517(V)} + IB (131-218)] + RBP (17-192) | mkwvtfisllflfssaysLVEEPQNLIKQNCELFEQLGEYKFQNALLVRYTKKVPEVSTPTLVEVSRNLGKVGSKCCKHPEAKRMPCAEDYLSVVLNQLCVLHEKTPVSDRVTKCCTESLVNRRPCFSALEVDDNPNLPRLVRPEVDVMCTAFHDNEETFLKKYLYEIARRHPYFYAPELLFFAKRYKAAFTECCQAADKAACLLPKLDELRDEGKASSAERDCRVSSFRVKENFDKARFSGTWYAMAKKDPEGLFLQDNIVAEFSVDETGQMSATAKGRVRLLNNWDVCADMVGTFTDTEDPAKFKMKYWGVASFLQKGNDDHWIVDTDYDTYAVQYSCRLLNLDGTCADSYSFVFSRDPNGLPPEAQKIVRQRQEELCLARQYRLIVHNGYCDG |

(continued)

| SEQ ID NO | Fusion protein | Amino acid sequence |
|---|---|---|
| 8 | Fusion protein 6: albumin [signal peptide (1-18) + IIIA {404-517(V)} + IB (134-218)] + RBP (17-192) | mkwvtfisllflflfssaysLVEEPQNLIKQNCELFEQLGEYKFQNALLVRYTKKVPEV STPTLVEVSRNLGKVGSKCCKHPEAKRMPCAEDYLSVVLNQLCVLHEKTP VSDRVTKCCTESLVNRRPCFSALEVPNLPRLVRPEVDVMCTAFHDNEETF LKKYLYEIARRHPYFYAPELLFFAKRYKAAFTECCQAADKAACLLPKLDEL RDEGKASSAERDCRVSSFRVKENFDKARFSGTWYAMAKKDPEGLFLQD NIVAEFSVDETGQMSATAKGRVRLLNNWDVCADMVGTFTDTEDPAKFKM KYWGVASFLQKGNDDHWIVDTDYDTYAVQYSCRLLNLDGTCADSYSFVF SRDPNGLPPEAQKIVRQRQEELCLARQYRLIVHNGYCDG |

2-2. Preparation of fusion protein

After preparing polynucleotides encoding the designed fusion proteins 1-6, recombinant expression vectors were prepared by cloning each DNA fragment into a XbaI/KpnI cut pcDNA3.1 (+) vector. Detailed information of the DNA fragments that encode the fusion proteins is summarized in Table 2. Transformants expressing the fusion proteins were prepared by introducing each vector into CHO cells using Lipofectamine 2000 (Invitrogen, Carlsbad, CA). The fusion proteins expressed and secreted by culturing the transformants were used after purifying by affinity chromatography and HPLC.

[Table 2]

| SEQ ID NO | Fusion protein-encoding polynucleotide | Sequence |
|---|---|---|
| 9 | Fusion protein 1 | CCGCTCTGGTGGAGCTGGTGAAGCATAAGCCTAAGGCTACAAA GGAGCAGCTGAAGGCCGTGATGGACGATTTCGCTGCCTTTGTG GAGAAGTGCTGTAAGGCTGACGATAAGGAGACCTGTTTCGCCG AGGAGGGCAAGAAGCTGGCGGCT |
| 10 | Fusion protein 2 | TGGATGGCACCTGTGCCGACAGCTATTCTTTCGTGTTTTCCCGC GATCCTAATGGCCTGCCCCCTGAGGCTCAGAAGATCGTGAGGC AGAGGCAGGAGGAGCTGTGCCTGGCCAGGCAGTACCGGCTGA TCGTGCATAATGGCTATTGTGACGGC |
| 11 | Fusion protein 3 | atgagatcgccagacgccatccctactttttatgctcctgagctgctgttctttgccaagcggtaca aggctgccttcacagagtgctgtcaggctgctgacaaggctgcttgcctgctgccaaagctgga tgagctgagggacgagggcaaggcttcttccgcc |

(continued)

| SEQ ID NO | Fusion protein-encoding polynucleotide | Sequence |
|---|---|---|
| 12 | Fusion protein 4 | ccagacgccatccctacttttatgctcctgagctgctgttctttgccaagcggtacaaggctgcctt cacagagtgctgtcaggctgctgacaaggctgcttgcctgctgccaaagctggatgagctgag ggacgagggcaaggcttcttccgcc |
| 13 | Fusion protein 5 | GACTGCTGAACCTGGATGGCACATGTGCTGACTCTTATTCCTTC GTGTTTTCTCGCGATCCAAATGGCCTGCCCCCTGAGGCCCAGA AGATCGTGAGGCAGAGGCAGGAGGAGCTGTGCCTGGCTAGGC AGTACCGGCTGATCGTGCATAATGGCTATTGTGACGGC |
| 14 | Fusion protein 6 | ACCTGGATGGCACATGTGCTGACTCTTATTCCTTCGTGTTTTCTC GCGATCCAAATGGCCTGCCCCCTGAGGCCCAGAAGATCGTGAG GCAGAGGCAGGAGGAGCTGTGCCTGGCTAGGCAGTACCGGCT GATCGTGCATAATGGCTATTGTGACGGC |

### 3. Quantitative real-time PCR

Total RNAs were prepared using TRIzol (Ambion, Austin, TX, USA) and cDNAs were constructed. qRT-PCR was conducted using the ABI QuantStudio™ 3 real-time PCR system. The PCR products were normalized to the mRNA level of GAPDH (glyceraldehyde 3-phosphate dehydrogenase).

### 4. Preparation of liver fibrosis animal model

A mouse model of liver fibrosis was prepared by inducing liver damage by intraperitoneally injecting $CCl_4$ dissolved in mineral oil at 1:1 to BALB/c mice at a concentration of 1 mL/kg for 6 weeks, 3 times a week. Only mineral oil of the same amount was administered to a control group. The mice were sacrificed 48 hours after the final injection of the $CCl_4$.

For evaluation of the fibrosis of the sacrificed mice, liver was taken out and tissue sections were prepared by fixing the liver tissue of each test group in 10% buffered formalin and embedding in paraffin. For histological analysis, the tissue sections were stained with sirius red and then observed with an optical microscope.

### 5. Statistical analysis

The results were expressed as mean $\pm$ standard deviation (SD). Statistical analysis was conducted by t-test. $P < 0.05$ was considered significant.

### [Experimental result]

### Example 1. Comparison of effect of fusion proteins 1 and 2 with existing albumin-RBP fusion proteins

The effect of the fusion proteins 1 and 2 designed in Example 2 was compared with that of RBP-albumin III and albumin III-RBP, which showed superior effect of inhibiting the activation of stellate cells or deactivating the same in the previous research. Specifically, after treating activated mouse hepatic stellate cells with 0.25 μM of each fusion protein for 20 hours, the expression level of α-SMA and collagen I was measured by real-time PCR. The existing fusion proteins (RBP-albumin III and albumin III-RBP) are the R-III and III-R fusion proteins of KR 10-1395394.

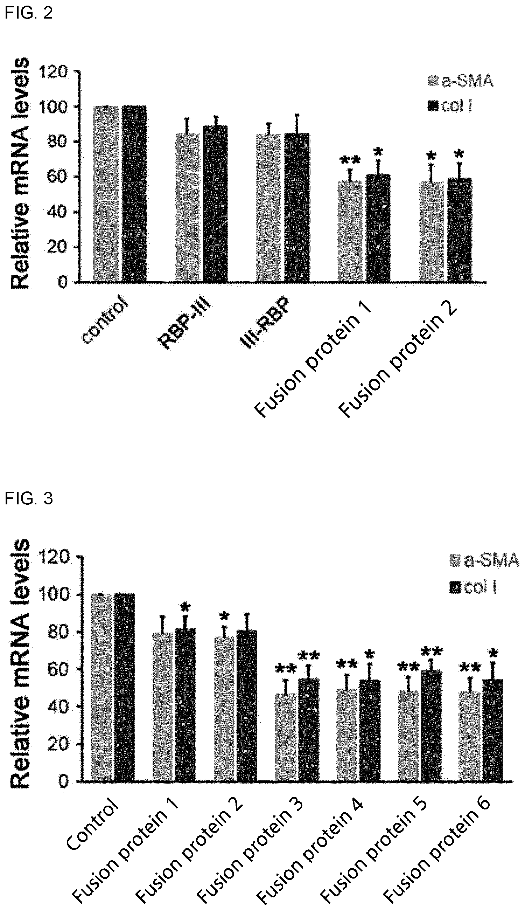

As a result, it was confirmed that the fusion proteins 1 and 2 decreased the mRNA expression of α-SMA and collagen I as compared to the existing fusion proteins, as shown in FIG. 2.

**Example 2. Comparison of effect of fusion proteins 1-6**

The effect of the fusion proteins 1 and 2, which showed superior effect of deactivating activated stellate cells as compared to the existing fusion proteins, was compared with that of the fusion proteins 3-6. Activated stellate cells were treated with the fusion proteins in the same manner as in Example 1 at a concentration of 0.125 $\mu$M.

As a result, it was confirmed that the fusion proteins 3-6 can significantly reduce the expression of $\alpha$-SMA and collagen I with smaller protein contents, as shown in FIG. 3.

**Example 3. Comparison of effect of fusion proteins 1 and 4 with existing albumin-RBP fusion proteins *in vivo***

The therapeutic effect of the fusion proteins was compared using a liver fibrosis induced by carbon tetrachloride (CCl$_4$) injection. Specifically, after intraperitoneally injecting carbon tetrachloride dissolved in mineral oil at 1:1 to BALB/c mice at a concentration of 1 mL/kg for 6 weeks, 3 times a week, the fusion protein (25, 12.5, 6.25 or 3 $\mu$g) or saline was intravenously administered for 2 weeks after the final injection of the CCl$_4$, 3 times a week (n = 5).

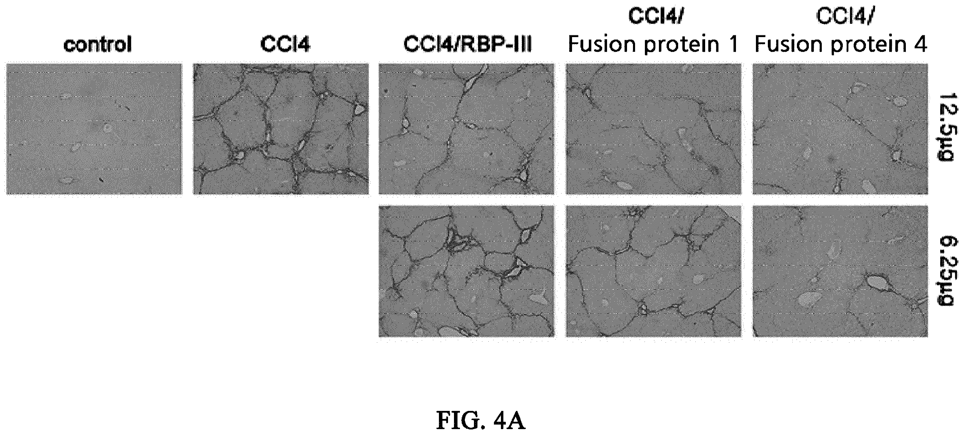

As a result of observing tissues with an optical microscope, the mice of a control group to which only mineral oil was administered showed normal liver structure, whereas the mice treated with CCl$_4$ showed severe liver tissue damage and fibrosis. In addition, it was confirmed that the administration of the albumin-RBP fusion proteins resulted in alleviation of fibrosis (FIG. 4A).

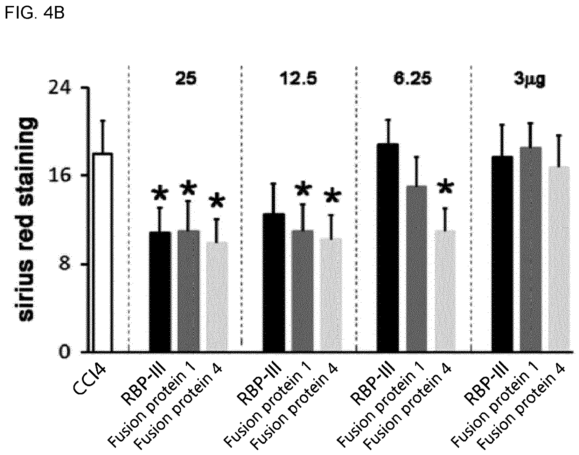

As a result of quantitative analysis of siruis red staining using the ImageJ software (NIH), the effect of alleviating fibrosis was different depending on the samples (FIG. 4B). Whereas the existing fusion protein RBP-albumin III showed significant effect only in the 25 $\mu$g administration group, the fusion protein 4 showed superior effect also in the 6.25 $\mu$g administration group. From this result, it was confirmed that the fusion proteins 1 and 4 exhibit superior effect at low concentrations as compared to the existing fusion proteins.

While the specific exemplary embodiments of the present disclosure have been described in detail, it will be obvious to those having ordinary knowledge in the art that they are merely specific exemplary embodiments and the scope of the present disclosure is not limited by them. Accordingly, it is to be understood that the substantial scope of the present disclosure is defined by the appended claims and their equivalents.

[Industrial Applicability]

A fusion protein of the present disclosure is expected to be a general-purpose platform technology capable of remarkably reducing the dose and frequency of administration of protein therapeutic agents to the human body.

**Claims**

1. A fusion protein comprising an amino acid sequence of SEQ ID NO 1 or 2.

2. A fusion protein comprising any amino acid sequence of SEQ ID NOS 3-8.

3. A polynucleotide encoding the fusion protein of claim 2, wherein the polynucleotide comprises any base sequence of SEQ ID NOS 9-14.

4. A recombinant vector comprising the polynucleotide of claim 3.

5. A transformant comprising the recombinant vector of claim 4.

6. A method for producing a fusion protein, comprising:

(1) a step of preparing a recombinant vector comprising a gene encoding a fusion protein comprising any amino acid sequence of SEQ ID NOS 3-8;
(2) a step of preparing a transformant by introducing the recombinant vector into a host cell;
(3) a step of culturing the transformant; and
(4) a step of obtaining a fusion protein from the cultured transformant.

7. A pharmaceutical composition for preventing or treating a fibrotic disease, comprising a fusion protein comprising any amino acid sequence of SEQ ID NOS 1-8.

8. The pharmaceutical composition according to claim 7, wherein the fibrotic disease is one or more disease selected from a group consisting of liver fibrosis, chronic hepatitis, cirrhosis, hepatic cancer, chemotherapy-associated steatohepatitis (CASH), lung fibrosis, renal fibrosis, renal failure, pancreatic fibrosis, chronic pancreatitis and pancreatic cancer.

9. A method for preventing or treating a fibrotic disease, comprising a step of administering a fusion protein comprising any amino acid sequence of SEQ ID NOS 1-8 to a subject.

10. A use of a fusion protein comprising any amino acid sequence of SEQ ID NOS 1-8 for preparation of a medication for preventing or treating a fibrotic disease.

FIG. 1

FIG. 2

FIG. 3

FIG. 4A

FIG. 4B

| INTERNATIONAL SEARCH REPORT | International application No. |
|---|---|
| | **PCT/KR2021/003959** |

**A. CLASSIFICATION OF SUBJECT MATTER**

**C07K 14/47**(2006.01)i; **C07K 14/765**(2006.01)i; **A61K 38/00**(2006.01)i; **A61P 43/00**(2006.01)i; **A61K 38/38**(2006.01)i

According to International Patent Classification (IPC) or to both national classification and IPC

**B. FIELDS SEARCHED**

Minimum documentation searched (classification system followed by classification symbols)

C07K 14/47(2006.01); C07K 1/00(2006.01); C07K 14/76(2006.01); C07K 19/00(2006.01); C12P 21/04(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 알부민(albumin), 레티놀 결합 단백질(retinol binding protein, RBP), 융합 단백질(fusion protein), 폴리뉴클레오티드(polynucleotide), 벡터(vector), 형질전환(transformation), 생산(production), 섬유 질환 (fibrosis disease)

**C. DOCUMENTS CONSIDERED TO BE RELEVANT**

| Category* | Citation of document, with indication, where appropriate, of the relevant passages | Relevant to claim No. |
|---|---|---|
| A | KR 10-2012-0101617 A (KOREA UNIVERSITY RESEARCH AND BUSINESS FOUNDATION) 14 September 2012 (2012-09-14)<br>See abstract, claims 1, 4-5 and 16, and SEQ ID NOs: 10-11. | 1-8,10 |
| A | LEE, H. et al. Fusion protein of retinol-binding protein and albumin domain III reduces liver fibrosis. EMBO Molecular Medicine. 2015, vol. 7, no. 6, pp. 819-830.<br>See entire document. | 1-8,10 |
| A | CHOI, S. et al. Recombinant fusion protein of albumin-retinol binding protein inactivates stellate cells. Biochemical and Biophysical Research Communications. 2012, vol. 418, pp. 191-197.<br>See entire document. | 1-8,10 |
| A | PARK, S. et al. Retinol binding protein-albumin domain III fusion protein deactivates hepatic stellate cells . Molecules and Cells. 2012, vol. 34, pp. 517-522.<br>See entire document. | 1-8,10 |
| A | EP 2277889 A2 (HUMAN GENOME SCIENCES, INC. et al.) 26 January 2011 (2011-01-26)<br>See entire document. | 1-8,10 |

☐ Further documents are listed in the continuation of Box C.  ☑ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "D" | document cited by the applicant in the international application | "X" document of particular relevance; the claimed invention cannot be considered novel or cannot be considered to involve an inventive step when the document is taken alone |
| "E" | earlier application or patent but published on or after the international filing date | |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" document member of the same patent family |
| "P" | document published prior to the international filing date but later than the priority date claimed | |

| Date of the actual completion of the international search | Date of mailing of the international search report |
|---|---|
| **15 July 2021** | **15 July 2021** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

International application No.

**PCT/KR2021/003959**

| Box No. II | Observations where certain claims were found unsearchable (Continuation of item 2 of first sheet) |

This international search report has not been established in respect of certain claims under Article 17(2)(a) for the following reasons:

1. ☑ Claims Nos.: **9**
   because they relate to subject matter not required to be searched by this Authority, namely:

   Claim 9 pertains to a method for treatment of the human body by surgery or therapy, as well as a diagnostic method (PCT 17(2)(a)(i) and PCT Rule 39.1(iv)).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2019)

| Patent document cited in search report | | | Publication date (day/month/year) | Patent family member(s) | | | Publication date (day/month/year) |
|---|---|---|---|---|---|---|---|
| KR | 10-2012-0101617 | A | 14 September 2012 | EP | 2682406 | A2 | 08 January 2014 |
| | | | | EP | 2682406 | B1 | 09 May 2018 |
| | | | | US | 10064915 | B2 | 04 September 2018 |
| | | | | US | 2013-0338074 | A1 | 19 December 2013 |
| | | | | US | 2016-0200796 | A1 | 14 July 2016 |
| | | | | US | 2017-0274044 | A1 | 28 September 2017 |
| | | | | US | 9273116 | B2 | 01 March 2016 |
| | | | | US | 9701732 | B2 | 11 July 2017 |
| | | | | WO | 2012-118323 | A2 | 07 September 2012 |
| | | | | WO | 2012-118323 | A3 | 13 December 2012 |
| EP | 2277889 | A2 | 26 January 2011 | CN | 101287750 | A | 15 October 2008 |
| | | | | CN | 102816241 | A | 12 December 2012 |
| | | | | CN | 102816241 | B | 22 July 2015 |
| | | | | CN | 1980687 | A | 13 June 2007 |
| | | | | CN | 1980687 | B | 13 May 2015 |
| | | | | EP | 1463751 | A2 | 06 October 2004 |
| | | | | EP | 1463751 | B1 | 22 May 2013 |
| | | | | EP | 1463752 | A2 | 06 October 2004 |
| | | | | EP | 1594530 | A2 | 16 November 2005 |
| | | | | EP | 1729795 | A2 | 13 December 2006 |
| | | | | EP | 1729795 | B1 | 03 February 2016 |
| | | | | EP | 1924596 | A2 | 28 May 2008 |
| | | | | EP | 1997829 | A1 | 03 December 2008 |
| | | | | EP | 2211888 | A1 | 04 August 2010 |
| | | | | EP | 2261250 | A1 | 15 December 2010 |
| | | | | EP | 2261250 | B1 | 01 July 2015 |
| | | | | EP | 2277888 | A2 | 26 January 2011 |
| | | | | EP | 2277888 | A3 | 27 April 2011 |
| | | | | EP | 2277889 | A3 | 27 April 2011 |
| | | | | EP | 2277889 | B1 | 09 July 2014 |
| | | | | EP | 2277910 | A1 | 26 January 2011 |
| | | | | EP | 2990417 | A1 | 02 March 2016 |
| | | | | JP | 2005-514060 | A | 19 May 2005 |
| | | | | JP | 2006-176514 | A | 06 July 2006 |
| | | | | JP | 2007-522806 | A | 16 August 2007 |
| | | | | JP | 2009-213477 | A | 24 September 2009 |
| | | | | JP | 2009-504157 | A | 05 February 2009 |
| | | | | JP | 2011-015690 | A | 27 January 2011 |
| | | | | JP | 2011-501959 | A | 20 January 2011 |
| | | | | JP | 2012-140448 | A | 26 July 2012 |
| | | | | JP | 2013-172734 | A | 05 September 2013 |
| | | | | JP | 2013-208118 | A | 10 October 2013 |
| | | | | JP | 2014-043448 | A | 13 March 2014 |
| | | | | JP | 2014-079245 | A | 08 May 2014 |
| | | | | JP | 2015-120714 | A | 02 July 2015 |
| | | | | JP | 2015-126746 | A | 09 July 2015 |
| | | | | JP | 5010923 | B2 | 29 August 2012 |
| | | | | JP | 5424521 | B2 | 26 February 2014 |
| | | | | JP | 5568582 | B2 | 06 August 2014 |
| | | | | JP | 5705418 | B2 | 22 April 2015 |

Form PCT/ISA/210 (patent family annex) (July 2019)

**INTERNATIONAL SEARCH REPORT**
Information on patent family members

International application No.

**PCT/KR2021/003959**

| Patent document cited in search report | Publication date (day/month/year) | Patent family member(s) | | | Publication date (day/month/year) |
|---|---|---|---|---|---|
| | | JP | 5829245 | B2 | 09 December 2015 |
| | | JP | 5856131 | B2 | 09 February 2016 |
| | | KR | 10-1184391 | B1 | 14 March 2013 |
| | | KR | 10-1271635 | B1 | 12 June 2013 |
| | | KR | 10-2007-0016119 | A | 07 February 2007 |
| | | KR | 10-2008-0071119 | A | 01 August 2008 |
| | | KR | 10-2012-0126065 | A | 20 November 2012 |
| | | US | 2005-0054570 | A1 | 10 March 2005 |
| | | US | 2005-0186664 | A1 | 25 August 2005 |
| | | US | 2006-0014254 | A1 | 19 January 2006 |
| | | US | 2006-0194735 | A1 | 31 August 2006 |
| | | US | 2006-0276396 | A1 | 07 December 2006 |
| | | US | 2006-0286635 | A1 | 21 December 2006 |
| | | US | 2007-0027306 | A1 | 01 February 2007 |
| | | US | 2007-0048282 | A1 | 01 March 2007 |
| | | US | 2007-0244047 | A1 | 18 October 2007 |
| | | US | 2007-0259815 | A1 | 08 November 2007 |
| | | US | 2008-0004206 | A1 | 03 January 2008 |
| | | US | 2008-0146503 | A1 | 19 June 2008 |
| | | US | 2008-0153751 | A1 | 26 June 2008 |
| | | US | 2008-0161243 | A1 | 03 July 2008 |
| | | US | 2008-0167238 | A1 | 10 July 2008 |
| | | US | 2008-0167239 | A1 | 10 July 2008 |
| | | US | 2008-0167240 | A1 | 10 July 2008 |
| | | US | 2008-0194481 | A1 | 14 August 2008 |
| | | US | 2008-0213886 | A1 | 04 September 2008 |
| | | US | 2008-0293629 | A1 | 27 November 2008 |
| | | US | 2009-093402 | A1 | 09 April 2009 |
| | | US | 2009-099073 | A1 | 16 April 2009 |
| | | US | 2010-0048472 | A1 | 25 February 2010 |
| | | US | 2010-0093627 | A1 | 15 April 2010 |
| | | US | 2010-0291033 | A1 | 18 November 2010 |
| | | US | 2011-0002888 | A1 | 06 January 2011 |
| | | US | 2011-0009312 | A1 | 13 January 2011 |
| | | US | 2012-0046221 | A1 | 23 February 2012 |
| | | US | 2013-0150296 | A1 | 13 June 2013 |
| | | US | 2013-0157933 | A1 | 20 June 2013 |
| | | US | 2014-0179596 | A1 | 26 June 2014 |
| | | US | 2014-0303077 | A1 | 09 October 2014 |
| | | US | 2014-0328794 | A1 | 06 November 2014 |
| | | US | 2015-0203568 | A1 | 23 July 2015 |
| | | US | 2015-0329619 | A1 | 19 November 2015 |
| | | US | 2016-0152687 | A1 | 02 June 2016 |
| | | US | 2017-096472 | A1 | 06 April 2017 |
| | | US | 7141547 | B2 | 28 November 2006 |
| | | US | 7189690 | B2 | 13 March 2007 |
| | | US | 7238660 | B2 | 03 July 2007 |
| | | US | 7238667 | B2 | 03 July 2007 |
| | | US | 7521424 | B2 | 21 April 2009 |
| | | US | 7569384 | B2 | 04 August 2009 |

Form PCT/ISA/210 (patent family annex) (July 2019)

**INTERNATIONAL SEARCH REPORT**
Information on patent family members

International application No.

**PCT/KR2021/003959**

| Patent document cited in search report | Publication date (day/month/year) | Patent family member(s) | | | Publication date (day/month/year) |
|---|---|---|---|---|---|
| | | US | 7592010 | B2 | 22 September 2009 |
| | | US | 7766274 | B1 | 03 August 2010 |
| | | US | 7799759 | B2 | 21 September 2010 |
| | | US | 7847079 | B2 | 07 December 2010 |
| | | US | 7977306 | B2 | 12 July 2011 |
| | | US | 8012464 | B2 | 06 September 2011 |
| | | US | 8071539 | B2 | 06 December 2011 |
| | | US | 8143026 | B2 | 27 March 2012 |
| | | US | 8205822 | B1 | 26 June 2012 |
| | | US | 8211439 | B2 | 03 July 2012 |
| | | US | 8252739 | B2 | 28 August 2012 |
| | | US | 8287859 | B2 | 16 October 2012 |
| | | US | 8513189 | B2 | 20 August 2013 |
| | | US | 8899513 | B1 | 02 December 2014 |
| | | US | 8993517 | B2 | 31 March 2015 |
| | | US | 9221896 | B2 | 29 December 2015 |
| | | US | 9296809 | B2 | 29 March 2016 |
| | | WO | 03-059934 | A2 | 24 July 2003 |
| | | WO | 03-060071 | A2 | 24 July 2003 |
| | | WO | 2003-059934 | A3 | 26 February 2004 |
| | | WO | 2003-060071 | A3 | 26 February 2004 |
| | | WO | 2005-003296 | A2 | 13 January 2005 |
| | | WO | 2005-003296 | A3 | 21 April 2005 |
| | | WO | 2005-077042 | A2 | 25 August 2005 |
| | | WO | 2005-077042 | A3 | 30 November 2006 |
| | | WO | 2007-021494 | A2 | 22 February 2007 |
| | | WO | 2007-021494 | A3 | 26 July 2007 |
| | | WO | 2009-058322 | A1 | 07 May 2009 |

Form PCT/ISA/210 (patent family annex) (July 2019)

**REFERENCES CITED IN THE DESCRIPTION**

**Patent documents cited in the description**

- KR 101395394